# EUROPÄISCHE PATENTSCHRIFT

(11) **EP 0 975 256 B1**
(45) Veröffentlichungstag und Bekanntmachung des Hinweises auf die Patenterteilung: **07.04.2004**
(21) Anmeldenummer: 98922676.6
(22) Anmeldetag: 09.04.1998
(51) Int. Cl.: A61B 1/00, A61B 1/04

(54) **KUPPLUNG ZUM MECHANISCHEN, LICHTLEITENDEN UND BILDLEITENDEN VERBINDEN EINES ENDOSKOPES MIT EINEM KAMERAMODUL**
COUPLING FOR MECHANICALLY AND OPTICALLY CONNECTING AN ENDOSCOPE TO A CAMERA MODULE SO AS TO TRANSMIT IMAGES FROM THE ENDOSCOPE TO THE CAMERA MODULE
RACCORD POUR RELIER MECANIQUEMENT ET OPTIQUEMENT UN ENDOSCOPE A UN MODULE A CAMERA DE FA ON A TRANSMETTRE DES IMAGES DE L'ENDOSCOPE A LA CAMERA

(30) Priorität: 14.04.1997 DE 19715510
(43) Veröffentlichungstag der Anmeldung: 02.02.2000
(73) Patentinhaber: Karl Storz GmbH & Co. KG, 78532 Tuttlingen (DE)
(72) Erfinder: CHATENEVER, David, Santa Barbara, CA 93105 (US); IRION, Klaus, M., D-78576 Liptingen (DE); EHRHARDT, André, D-78573 Wurmlingen (DE); RUDISCHHAUSER, Jürgen, D-78532 Tuttlingen (DE); MATTSSON-BOZE, Daniel, Sacramento, CA 95835 (US)
(74) Vertreter: Weller, Wolfgang, Dr.
(86) Internationale Anmeldenummer: PCT/EP1998/002078
(87) Internationale Veröffentlichungsnummer: WO 1998/046117

(56) Entgegenhaltungen:
- EP-A- 0 501 088
- US-A- 5 239 983
- US-A- 5 498 230

## Beschreibung

Die Erfindung betrifft eine Kupplung zum mechanischen, lichtleitenden und bildleitenden Verbinden eines Endoskopes mit einem Kameramodul, wobei das Endoskop einen langen zylindrischen Schaft von etwa gleichbleibendem Durchmesser aufweist, wobei der Schaft ein Bildleitsystem und ein Lichtleitsystem aufnimmt.

Aus der US-A-5 498 230 ist eine Kupplung zum lichtleitenden und bildleitenden Verbinden eines Endoskopes E und einem Kameramodul bekannt, wobei die Kupplung aus zwei Kupplungsteilen besteht, die jeweils aus dem Kupplungsende des Endoskopes E und dem Kupplungsende des Kameramoduls bestehen, wobei am Kupplungsende des Endoskopes E zwei vorstehende Zapfen angebracht sind, nämlich ein Lichtleitzapfen F und ein darunterliegender dickerer bildleitender Zapfen. Am Kupplungsende des Kameramoduls sind entsprechende komplementäre Aufnahmen vorgesehen, in die die Zapfen eindringen.

Endoskope haben in der minimal-invasiven Chirurgie eine weite Verbreitung gefunden. Endoskope enthalten typischerweise ein Bildleitsystem, bspw. in Form eines im Schaft des Endoskopes angeordneten Stablinsensystems, wie es von der Anmelderin vertrieben wird. Das Bildleitsystem kann auch als geordnetes Glasfaserbündel ausgebildet sein. Um Licht an die Operationsstelle zu bringen, ist ferner ein Lichtleitsystem vorhanden, meist in Form von Glasfaserleitern.

Am proximalen Ende ist das Endoskop bzw. dessen Bildleitsystem üblicherweise mit einem Okular versehen. Das Lichtleitsystem führt meist rechtwinklig vom Bildleitsystem bzw. vom Okular am proximalen Ende ab, um über einen externen Lichtleiter mit einer Lichtquelle verbunden zu werden.

In einer Weiterentwicklung dieser Technologie ist es bekannt geworden, das proximale Ende des Endoskopes statt mit einem Okular mit einem Kameramodul zu versehen, das das vom Endoskop erzeugte Bild über eine Bildaufnehmereinheit in ein Videobild umsetzt, das bspw. auf einem Monitor wiedergegeben wird. Der Operateur kann in diesem Fall das vom Endoskop erzeugte Bild auf einem großflächigen Monitor beobachten.

Das Kameramodul, das an das proximale Ende des Endoskops angeschlossen wird, enthält meist einen sogenannten CCD-Sensor (Charge-Coupled-Device-Sensor) in Form eines lichtempfindlichen Chips, der die optischen Signale in elektrische Signale umsetzt, die vom bilderfassenden Kameramodul einem abseits gelegenen Bildverarbeitungssystem zugeführt werden.

Zur Erleichterung der Handhabung ist es bekannt geworden, das Kameramodul mit dem Endoskop zu koppeln, so daß bspw. zunächst das Endoskop über einen Trokar in den Körper an die Operationsstelle eingeführt wird und erst zur eigentlichen Operation das Kameramodul angeschlossen wird.

Somit besteht ein Bedarf, diese beiden Elemente bildleitend zu koppeln. Darüber hinaus müssen auch diese Elemente mechanisch miteinander verkoppelt werden, damit sich die Verbindung während der Operation oder während der Handhabung nicht löst. Zugleich muß eine lichtleitende Kupplung vorhanden sein, um das Endoskop bzw. dessen Lichtleitsystem mit einer Lichtquelle zu verbinden.

Von einem solchen Kupplungssystem wird nicht nur eine zuverlässige mechanische, lichtleitende und bildleitende Verbindung verlangt, sondern der Kupplungsvorgang soll möglichst ohne hohe Aufmerksamkeit, dennoch sicher durchgeführt werden können. Es muß daher dafür gesorgt werden, daß die Kupplungselemente von Endoskop einerseits und Kameramodul andererseits so aufeinander abgestimmt sind, daß diese passend ineinandergefügt werden und dies so durchgeführt wird, ohne daß Irrtümer stattfinden können und von dem Operateur keine hohe Aufmerksamkeit verlangt wird.

Darüber hinaus wird von einem solchen Kupplungssystem auch eine gewisse Flexibilität hinsichtlich anderer Anwendungen verlangt.

Wie eingangs erwähnt, wird bei manchen Operationstechniken zunächst das Endoskop unter direkter visueller Kontrolle an Ort und Stelle gebracht, ohne daß das Kameramodul angekoppelt ist. Da ein solches Endoskop keinen mechanischen Okularaufsatz besitzt, diese Funktion übernimmt ja das Kameramodul, ist es für den Operateur schwierig, das Endoskop an Ort und Stelle zu bringen, so daß es hilfreich wäre, für diesen Vorgang das Endoskop kurzfristig mit einem Okularaufsatz zu versehen.

Es wurden nun verschiedene Lösungswege eingeschlagen, um die mechanische, lichtleitende und bildleitende Kupplung bzw. Verbindung zwischen Endoskop und Kameramodul zu schaffen.

Eine bekannte Lösung, bei der für jeden der drei Kupplungsvorgänge (mechanisch, lichtleitend, bildleitend) eigene Kupplungsbauelemente vorgesehen sind, führt zu einer sperrigen Bauweise, bspw. bei der eingangs erwähnten Kupplung mit einer rechtwinklig zur optischen Achse abführenden Bildleitsystemführung.

Hier sind für die einzelnen zu koppelnden Systeme unterschiedliche Kupplungsrichtungen vorhanden, so daß eine erhöhte Aufmerksamkeit bei dem Verbinden notwendig ist und ein sperrig bauender Zusammenbau resultiert.

Es ist daher Aufgabe der vorliegenden Erfindung, ein Kupplungssystem vorzuschlagen, mit dem auf einfache, handhabungsfreundliche und sichere Art eine mechanische, lichtleitende und bildleitende Verbindung eines Endoskopes mit einem Kameramodul geschaffen werden kann, wobei dies mit baulich geringem Aufwand und geringem Bauraum erfolgen soll.

Die Erfindung wird durch eine Kupplung zum mechanischen, lichtleitenden und bildleitenden Verbinden eines Endoskopes mit einem Kameramodul gelöst, die aus zwei Kupplungsteilen besteht, nämlich aus einem Kupplungsende des Endoskopes und einem Kupplungsende des Kameramoduls, wobei das Endoskop einen langen zylindrischen Schaft von etwa gleichbleibendem Durchmesser aufweist, wobei der Schaft ein Bildleitsystem und ein Lichtleitsystem aufnimmt, wobei am Kupplungsende des Endokopes ein erster zylindrischer vorstehender Zapfen bestimmten Durchmessers und bestimmter Länge in Kupplungsrichtung angebracht ist, in dessen Innerem ein proximaler Endabschnitt des Lichtleitsystems aufgenommen ist, und am Kupplungsende des Endoskopes ein zweiter, etwa zylindrischer vorstehender Zapfen in Kupplungsrichtung angebracht ist, dessen Länge und Durchmesser größer als die Länge und der Durchmesser des ersten Zapfens sind, wobei im Innern des zweiten Zapfens ein proximaler Endabschnitt des Bildleitsystems aufgenommen ist, wobei der zweite Zapfen mit einer am Kameramodul angeordneten Verriegelung zum mechanischen Verriegeln der Kupplung zusammenwirkt, wobei sich erster und zweiter Zapfen im Abstand nebeneinander erstrecken, wobei am Kupplungsende des Kameramoduls den beiden Zapfen entsprechende komplementäre Aufnahmen vorgesehen sind, in die die Zapfen eindringen, und wobei ein Boden der Aufnahme, in die der zweite Zapfen eindringt, optisch mit dem Bildaufnahmesystem des Kameramoduls verbunden ist und die Aufnahme, in die der kürzere erste Zapfen aufnehmbar ist, einen Lichtleiter aufweist.

Diese Maßnahmen haben nun zahlreiche erhebliche Vorteile im Sinne der Lösung der gestellten Aufgabe.

Die mechanische, lichtleitende und bildleitende Kupplung ist durch einen einzigen einfachen linearen Verschiebevorgang durchzuführen, in dem nämlich die beiden Zapfen in die entsprechenden Aufnahmen des Kameramoduls eingeschoben werden. Aufgrund der Tatsache, daß nunmehr das Kameramodul eine Aufnahme für einen Lichtleiter aufweist, kann zugleich sowohl die lichtleitende als auch die bildleitende Verbindung durch ein Ineinanderstecken der Bauelemente aufgebaut werden. Aufgrund der Tatsache, daß einer der beiden Zapfen dicker und länger als der andere ist, ist ein versehentliches, also ein verkehrtes Einsetzen nicht möglich. Aufgrund der Tatsache, daß der dickere Zapfen auch gleichzeitig der längere ist, kann mit diesem dikkeren und längeren Zapfen, ohne besondere Aufmerksamkeit, die entsprechend größere Aufnahme am Kameramodul ertastet werden und dann durch eine Einschiebbewegung die Kupplung geschlossen werden. Fehlerhafte Ansätze sind dadurch nicht mehr möglich, da der dickere längere Zapfen nicht an der durchmessergeringeren Ausnehmung für den kleineren und kürzeren Zapfen angesetzt werden kann.

Zugleich wird bei diesem Einschieben die mechanische Verriegelung bzw. Kupplung bewerkstelligt. Daß dies nun mit dem größeren und dickeren Zapfen erfolgt, hat den erheblichen Vorteil, daß ein stabiler mechanischer Verbund mit einem stabilen Bauteil des Endoskopes, nämlich dem langen und durchmessergrößeren Zapfen bewerkstelligt wird. Dadurch können dann die mechanischen Kräfte, die auf die Kupplungsstelle einwirken, von einem sehr großen stabilen und kompakten Bauteil, nämlich dem großen und langen Zapfen aufgenommen werden, so daß eine auf Dauer mechanisch stabile Kupplung bewerkstelligt wird. Aufgrund der Tatsache, daß der größere Zapfen auch der längere Zapfen ist, und dieser das Bildleitsystem trägt, erfolgt der bildleitende Schluß in einem axialen Abstand zu lichtleitenden Schluß. Diese Maßnahme hat nun den Vorteil, daß eventuell an dem Lichtschluß austretendes Streulicht nicht unmittelbar mit der axial davon beabstandeten bildleitenden Verbindungsstelle in Verbindung treten kann. Damit sind die Nachteile der Verbindung von Bild und Licht auf gleicher Höhe oder bspw. bei koaxialer Anordnung beseitigt.

Es sind nunmehr nur noch zwei kompakte Kupplungsteile vorhanden, nämlich das Kupplungsende des Endoskopes einerseits mit den im Abstand und parallel zueinander angeordneten vorspringenden Zapfen und das Kameramodul mit den entsprechenden Aufnahmen, das außerdem noch den Lichtleiter trägt. Aufgrund der Tatsache, daß die beiden Zapfen nebeneinander und im Abstand angeordnet sind, ist es noch möglich, weitere Bauelemente in deren Nähe oder Umgebung anzuordnen, ohne diese Bauelemente zu beeinträchtigen. Der in das Kameramodul eingedrungene dickere und längere Zapfen, der das Bildleitsystem des Endoskopes trägt, kommt im Boden der Ausnehmung des Kameramoduls unmittelbar mit dem optischen Bildaufnahmesystem des Kameramoduls in optische Verbindung, wodurch eine hervorragende optische Übertragung des Bildes vom Endoskop auf das Kameramodul sichergestellt ist, und dies noch in einer relativ tief innenliegenden, also von den äußeren Einflüssen, auch von äußeren Lichteinflüssen geschützten Stelle der Kupplung. Dadurch, daß der Lichtleiter nun ebenfalls im Kameramodul aufgenommen ist, entfallen sperrige, seitlich abstehende Lichtleitersysteme.

Somit wird die Aufgabe durch die Gesamtheit dieser Merkmale vollkommen gelöst.

In einer weiteren Ausgestaltung der Erfindung stehen die beiden Zapfen von einem Gehäuse vor, dessen Querschnittskontur einer um die beiden Zapfen unterschiedlichen Durchmessers umlaufenden Ortslinie entspricht.

Diese Maßnahme hat nun den Vorteil, daß die sich verjüngende ovale Ausgestaltung des Gehäuses einfach durch den Tastsinn einer menschlichen Hand erfaßt und erkannt werden kann, so daß ohne das Endoskop visuell zu beobachten, die Handhabungsperson weiß, welcher der beiden Zapfen wo liegt. In Zusammenhang mit der Tatsache, daß der dickere Zapfen ohnehin länger ist, kann die exakte Lage des Endoskopes in der Hand ohne visuellen Kontakt ertastet werden.

In einer weiteren Ausgestaltung der Erfindung weist das Kameramodul im Kupplungsbereich ein Gehäuse auf, dessen Querschnittskontur einer um die beiden Aufnahmen unterschiedlichen Durchmessers umlaufenden Ortslinie entspricht.

Diese Maßnahme hat, wie zuvor in Zusammenhang mit der Ausgestaltung des Endoskopes erwähnt, den Vorteil, daß durch den Tastsinn einer menschlichen Hand diese ovale asymmetrische Kontur einfach erfaßt und erkannt werden kann, so daß ebenfalls ohne visuelle Kontrolle die exakte Lage des Kameramoduls in der Hand erkannt wird.

In Kombination dieser beiden zuvor erwähnten Ausgestaltungen kann also die Bedienungsperson bspw. mit einer Hand das Endoskop und dessen genaue Ergreiflage im Kupplungsbereich erfassen, mit der anderen Hand das Kameramodul und ebenfalls dessen Ergreiflage einfach erfassen, so daß dann die beiden zu kuppelnden Elemente ohne visuellen Kontakt ineinandergesteckt werden können. Dies erleichtert erheblich die Handhabung, insbesondere wenn während einer Operation rasch ein Kameramodul gegen ein anderes oder ein Kameramodul mit einem anderen Endoskop, das ebenfalls bei der Operation verwendet wird, gekoppelt werden muß.

In einer weiteren Ausgestaltung der Erfindung ist die Verriegelung als ein quer zur Kupplungsrichtung verschiebbarer Riegel ausgebildet, der in eine Aussparung am zweiten Zapfen einrückbar ist.

Diese Maßnahme hat den Vorteil, daß zum Schließen und/oder Lösen der Kupplung der Riegel quer zur Kupplungsrichtung verschoben wird und in die Aussparung am zweiten Zapfen ein- bzw. ausrückt. Dies sind alles Vorgänge, die ohne visuellen Kontakt mit den Fingern einer Hand gesteuert werden können und wobei das Ein- bzw. Ausrasten des Riegels in die Aussparung am Zapfen der Bedienungsperson anzeigt, ob die Kupplung geschlossen oder geöffnet ist. Muß der Riegel bspw. zum Schließen der Kupplung in die Aussparung eingedrückt werden, kann dies durch entsprechendes Betätigen des Riegels mit einem Finger bewerkstelligt werden, die exakte Riegelung kann dadurch festgestellt werden, daß der große Zapfen nicht mehr bewegt bzw. nicht mehr abgezogen werden kann. Umgekehrt kann das ebenfalls gleich festgestellt werden, wenn bspw. der Riegel zum Lösen der Kupplung gedrückt werden muß. Dies wird dann möglich, wenn der große Zapfen außer Eingriff mit dem Riegel kommt und vom Kameramodul abgezogen werden kann. Hier wirkt sich erneut der Vorteil aus, daß die mechanische Kupplung mit dem relativ großen und langen Zapfen erfolgt, der dann auch die Kräfte, die beim Lösen oder noch nicht ganz Verriegeln der Kupplung auftreten, aufnehmen kann.

Es sind zahlreiche Ausgestaltungen des Riegels denkbar, z.B. als Kugelrasten, Haken, Rastnasen oder dgl.

In einer weiteren Ausgestaltung der Erfindung ist der Riegel durch die Kraft einer Feder beaufschlagt und ragt etwas in die Aufnahme für den zweiten Zapfen radial hinein.

Diese Ausgestaltung hat den Vorteil, daß zum einen durch die Kraft der Feder der Riegel in eine ganz definierte Position gedrückt wird, sinnvollerweise in die schließende Position, und dadurch, daß der Riegel etwas in die Aufnahme hineinragt, der dickere Zapfen nur dann vollständig in die Aufnahme eingeschoben bzw. von dieser abgezogen werden kann, wenn der Riegel herausbewegt worden ist. Dies sind alles Vorgänge, die durch den Tastsinn der Hand erfaßt und gesteuert werden können, so daß keine visuelle Aufmerksamkeit bzw. Beobachtung beim Schließen und der Lösen der Kupplung notwendig ist. Dadurch wird weiter erheblich die Handhabungsfreundlichkeit erhöht.

In einer weiteren Ausgestaltung der Erfindung weist der zweite Zapfen endseitig einen konischen Abschnitt auf, der von einer Hinterschneidung gefolgt ist.

Diese Maßnahme hat nun den Vorteil, daß durch den konischen Abschnitt eine Einschubhilfe beim Einschieben des Zapfens in die Ausnehmung gegeben ist, so daß schon durch ein ungefähres Ansetzen ein exaktes Einschieben gewährleistet ist. Zugleich kann die konische Fläche dazu herangezogen werden, den Riegel beim Einschieben radial zu verschieben.

In einer weiteren Ausgestaltung der Erfindung ist die Hinterschneidung im zweiten Zapfen als Ringnut ausgebildet.

Diese Maßnahme hat den Vorteil, daß eine relativ große Eingriffsfläche mit dem Riegel möglich ist, so daß die auf die Kupplung einwirkenden mechanischen Kräfte großflächig abgeleitet werden können, was der Lebensdauer und der mechanischen Stabilität zuträglich ist.

In einer weiteren Ausgestaltung der Erfindung steht der Riegel über das Gehäuse des Kameramoduls vor.

Diese Maßnahme hat den Vorteil, daß der Riegel bspw. durch den Finger einer Hand einfach ertastet und betätigt werden kann, ohne daß dazu ein spezielles Werkzeug oder eine hohe Aufmerksamkeit notwendig ist.

In einer weiteren Ausgestaltung der Erfindung ist im Gehäuse des Endoskopes, von dem die Zapfen vorstehen, ein Zwischenabbildungssystem aufgenommen.

Diese Maßnahme hat nun einen erheblichen Vorteil im Hinblick auf die Standardisierung der Kupplung. Es sind Endoskope unterschiedlichen Schaftdurchmessers im Einsatz, d.h. sehr dünne Endoskopschäfte im Bereich von etwa 1 mm bis zu Endoskopschäften von 10 mm. Dementsprechend können unterschiedliche Linsensysteme mit unterschiedlichen Durchmessern in diesen unterschiedlichen Endoskopen aufgenommen werden. Ist nun in dem Gehäuse, von dem die Zapfen vorstehen, das Zwischenabbildungssystem vorhanden, können unabhängig von dem Durchmesser des Schaftes am Ende des zweiten dickeren Zapfens entsprechend gewünschte Bilder, also Bilder bestimmter Größe oder bestimmten Bildausschnittes zur Verfügung gestellt werden. Anders ausgedrückt, kann dem Kameramodul ein und dieselbe Bildeinheit bzw. Bildeinheitsgröße vom Endoskop zugeführt werden, unabhängig davon, ob es sich um ein extrem dünnes oder dickes Endoskop mit unterschiedlichen Linsendurchmessern handelt. Dies erleichtert die Handhabbarkeit deshalb, da dann der Operateur nicht lang den Vergrößerungsmaßstab über das Bildverarbeitungssystem einstellen muß, abhängig davon, ob er nun gerade das Kameramodul an ein dünnes oder an ein dickes Endoskop angeschlossen hat.

In einer weiteren Ausgestaltung der Erfindung weist das Kameramodul ein Bildaufnahmesystem mit mindestens einem CCD-Sensor auf.

Diese an sich bekannte Maßnahme hat den Vorteil, daß das Kameramodul eine kleine kompakte Baueinheit darstellen kann und die raumergreifenden Bauelemente abseits der Kupplungsstelle liegen können.

In einer weiteren Ausgestaltung der Erfindung weist das Kameramodul eine Fokussiereinheit mit einem am Gehäuse angeordneten Stellglied auf.

Diese Maßnahme hat den Vorteil, daß unmittelbar im Bereich der Kupplung nach Bewerkstelligung der Kopplung über die Fokussiereinheit ein scharfes Bild eingestellt werden kann. Dies wird besonders durch das am Gehäuse angeordnete Stellglied erleichtert.

In einer weiteren Ausgestaltung der Erfindung bewirkt die Fokussiereinheit eine Scharfeinstellung des Bildes über eine Verstellung von Linsenelementen und/oder des CCD-Sensors.

Diese Maßnahme hat den Vorteil, daß mit einfachen Systemen die Scharfeinstellung des Bildes bewerkstelligt werden kann, wodurch dennoch eine kompakte und schlanke Bauweise erhalten bleibt.

In einer weiteren Ausgestaltung der Erfindung ist das Bildaufnahmesystem zur Bildaufrichtung drehbar im Gehäuse des Kameramoduls aufgenommen.

Unter Bildaufrichtung versteht man eine bestimmte Horizontal-Ausrichtung des vom Kameramodul erzeugten Monitorbildes nach Verdrehen des Endoskopes meist mit asymmetrischer Blickrichtung (z.B. 30°) um die Schaftachse. Manche Operateure wünschen eine gleichbleibende Horizontal-Ausrichtung des auf dem Monitor ersichtlichen Operationsbildes, wenn während einer Operation das Endoskop aus einer Ausgangsstellung verdreht wird. Dazu ist es an sich bekannt geworden, die Bildaufnehmereinheit entsprechend nachzudrehen, um dadurch das Bild wieder "aufzurichten".

Die nun vorgeschlagene Maßnahme, das drehbare Bildaufnahmesystem im Gehäuse des Kameramoduls im Bereich der Kupplung aufzunehmen, hat den erheblichen handhabungsmäßigen Vorteil, daß nach Schließen der Kupplung nicht nur das Bild durch Fokussieren scharfgestellt sondern auch in die gewünscht Position aufgerichtet werden kann, wobei diese Manipulationen alle mit ein und derselben Hand an der Kupplung durchgeführt werden können.

In einer weiteren Ausgestaltung der Erfindung erfolgt die Bildaufrichtung über eine Drehbarkeit des CCD-Sensors oder über eine Zwischenoptik, z.B. über sogenannte K-Prismen.

Diese Maßnahme hat den Vorteil, daß die Bildaufrichtung durch kompakt bauende Elemente direkt im Gehäuse des Kameramoduls bewerkstelligt werden können.

In einer weiteren Ausgestaltung der Erfindung weist der zweite Zapfen und die diesen aufnehmende Aufnahme jeweils ein Fenster auf.

Diese Maßnahme hat den Vorteil, daß durch mechanisch robuste und optisch durchlässige Mittel die bildleitende Verbindung zwischen dem Bildleitsystem des Endoskopes und der Kamera geschaffen werden kann. Das Fenster erlaubt einen dichten Abschluß des Bildleitsystems des Endoskopes, so daß dieses problemlos autoklaviert werden kann.

In einer weiteren Ausgestaltung der Erfindung ist sowohl im Endoskop als auch im Kameramodul zumindest eine weitere Kupplungsstelle für einen Insufflations- oder Spülkanal vorgesehen.

Diese Maßnahme hat nun den erheblichen Vorteil, daß mit der Kupplung auch zugleich eine Kupplung eines am Endoskop vorhandenen Spül- oder Insufflationskanals geschaffen werden kann. Dementsprechend ist dann ein entsprechender Kanal im Gehäuse des Kameramoduls vorgesehen.

In einer weiteren Ausgestaltung der Erfindung ist am Gehäuse des Endoskopes ein endoskopspezifisches Codierelement vorgesehen, das mit einem Leseelement am Kameramodul gekoppelt wird.

Diese Maßnahme hat den Vorteil, daß nach der Kopplung vom Kameramodul sofort erfaßt werden kann, mit welchem Endoskop (z.B. Blickrichtung) es gekoppelt worden ist, so daß dann bestimmte bildtechnische Voreinstellungen hinsichtlich Farbe, Filter, Bildausschnitt, Bildhelligkeit, -farbigkeit oder dgl. automatisch eingestellt werden können, was ebenfalls die Handhabbarkeit erleichtert. Das Codierelement kann als mechanisches, optisches oder elektromechanisches Element ausgeführt sein.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine Seitenansicht der beiden miteinander zu koppelnden Bauelemente, nämlich Endoskop und Kameramodul in nicht gekoppeltem Zustand;
- Fig. 2: einen Schnitt längs der Linie II-II in Fig. 1;
- Fig. 3: eine Draufsicht auf das stirnseitige proximale Ende des Endoskopes; und
- Fig. 4: einen Längsschnitt von Endoskop und Kameramodul von Fig. 1 in gekoppeltem System, wobei der Übersichtlichkeit halber die Bauelemente des Bild- und Lichtleitersystems weggelassen sind.

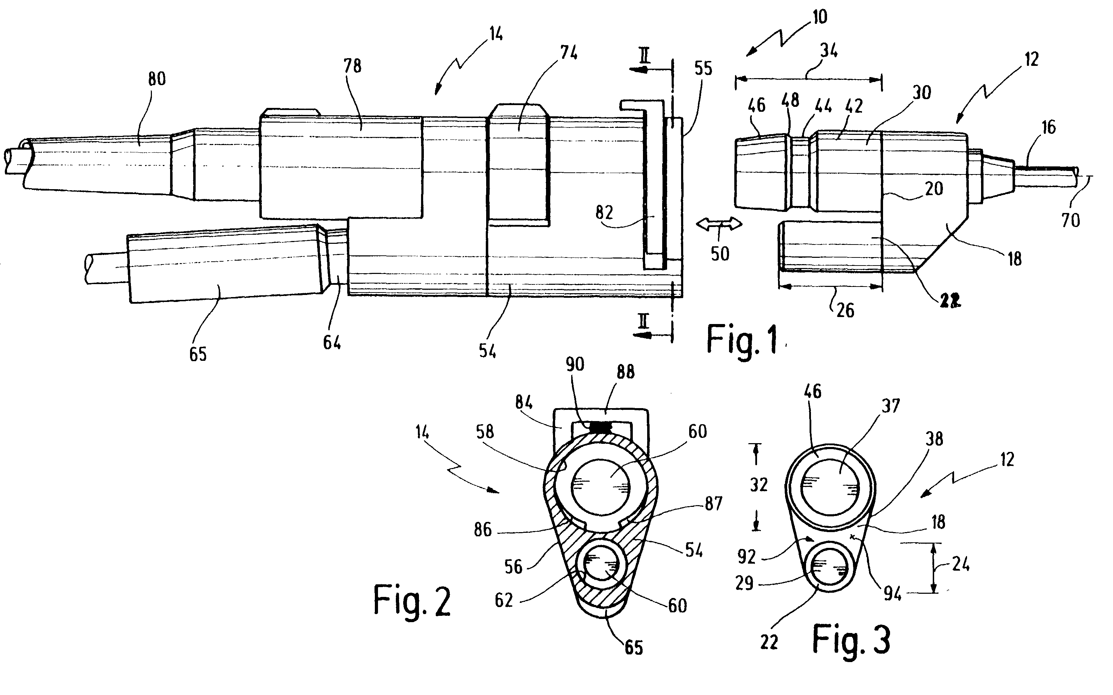

Ein in den Fig. 1 bis 4 in seiner Gesamtheit mit der Bezugsziffer 10 bezeichnetes Kupplungssystem weist ein Endoskop 12 auf, das mit einem Kameramodul 14 gekoppelt werden soll.

Das Endoskop 12 weist einen langerstreckten zylindrischen Schaft 16 auf, der proximal mit einem Gehäuse 18 versehen ist. Im Schaft 16 ist, wie das bei Endoskopen allgemein üblich ist, ein Lichtleitsystem und ein Bildleitsystem aufgenommen. An einem Kupplungsende 20 des Gehäuses 18 steht ein etwa zylindrischer erster Zapfen 22 vor, der einen Durchmesser 24 und eine Länge 26 aufweist.

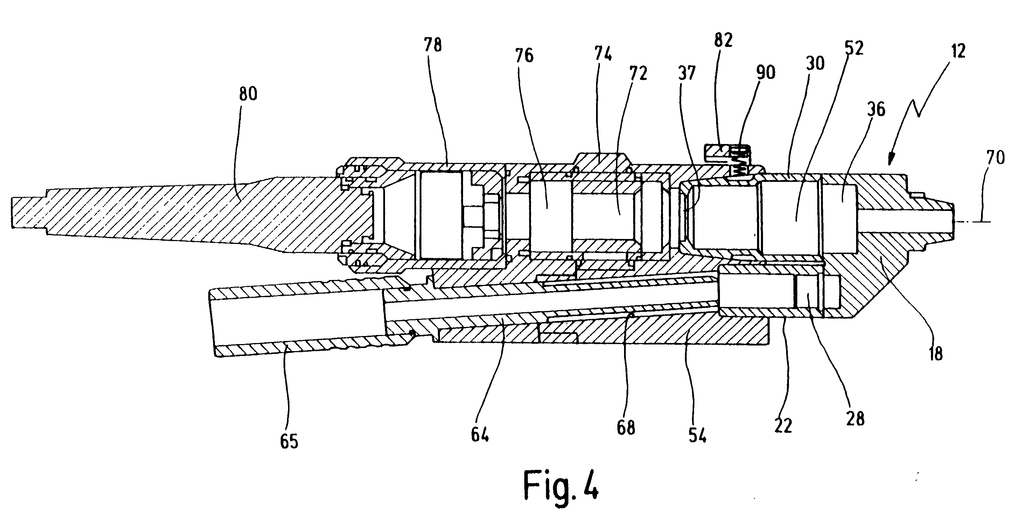

Im ersten Zapfen 22 ist der proximale Endabschnitt des Lichtleitsystems 28 (siehe Schnittdarstellung von Fig. 4) aufgenommen. Ein Fenster 29 bildet einen proximalen Abschluß des Zapfens 22.

Vom Kupplungsende 20 des Gehäuses 18 erstreckt sich ein zweiter etwa zylindrischer Zapfen 30 vor, der einen Durchmesser 32 und eine Länge 34 aufweist.

Wie aus der Darstellung von Fig. 2 zu erkennen, erstreckt sich der zweite Zapfen 30 parallel zum ersten Zapfen 22, jedoch im Abstand zu diesem. Die Länge 34 des zweiten Zapfens 30 ist größer als die Länge 26 des ersten Zapfens 22. Dasselbe gilt für den Durchmesser 32 des zweiten Zapfens 30.

Der zweite Zapfen 30 dient dazu, den proximalen Endabschnitt des Bildleitsystems 36 des Endoskopes 12 aufzunehmen. Ein proximaler Abschluß des zweiten Zapfens 30 bildet ein Fenster 37.

Wie insbesondere aus der Draufsicht von Fig. 3 zu erkennen, weist das Gehäuse 18 eine Querschnittskontur auf, die einer die beiden Zapfen 22 und 30 umrundenden ovalen asymmetrischen Ortslinie 38 entspricht. Wird also das Endoskop 12 im Bereich des Gehäuses 18 von einer Hand ergriffen, stellt man einen dickeren und einen dünneren Bereich fest.

Der zweite Zapfen 30 weist, ausgehend vom Gehäuse 18, einen zylindrischen Abschnitt 42, eine Ringnut 44 und einen endseitigen konischen Abschnitt 46 auf. Die dem konischen Abschnitt 46 zugeneigte Flanke der Ringnut 44 stellt zugleich eine Hinterschneidung 48 dar. Beide Zapfen 22 und 30 erstrecken sich in einer Kupplungsrichtung 50. Die Mittellängsachse des zweiten Zapfens 30 stellt zugleich die optische Achse 70 des Endoskopes und der Kupplung 10 dar.

y Wie insbesondere aus der Schnittdarstellung von Fig. 4 zu entnehmen, ist im Gehäuse 18 bzw. im zylindrischen Abschnitt 42 des Zapfens ein Zwischenabbildungssystem 52 aufgenommen. Dieses Zwischenabbildungssystem 52 dient dazu, um das vom Endoskopschaft 16 kommende Bild auf eine bestimmte gleiche standardisierte Größe am Fenster 37, das ebenfalls eine Standardgröße einnimmt, zu bringen.

Das mit dem Endoskop 12 zu kuppelnde Kameramodul 14 weist ein Gehäuse 54 auf, dessen umfängliche Querschnittskontur, wie das insbesondere aus Fig. 2 ersichtlich ist, eine asymmetrische ovale Ortslinie 56 umgrenzt, also einen ähnlichen Charakter wie beim Gehäuse 18 des Endoskopes 12. Auch dadurch wird bei Ergreifen des Gehäuses 54 von der Hand der Person der Eindruck eines dicken und eines dünnen Bereiches vermittelt.

Ausgehend von einem Kupplungsende 55 weist das Gehäuse 54 eine erste sacklochartige Aufnahme 58 auf, deren Boden über ein Fenster 60 verschlossen ist. Die Länge und der lichte Durchmesser der Aufnahme 58 ist so gewählt, daß darin der zweite Zapfen 30 passend aufgenommen werden kann. Neben der Aufnahme 58 ist noch eine weitere Aufnahme 62 vorgesehen, die, wie das insbesondere aus der Schnittdarstellung von Fig. 4 ersichtlich ist, als durchgehende Bohrung 68 ausgebildet ist.

In die Aufnahme 62 ist ein Lichtleiter 64 eingeschoben, dessen äußerstes Ende mit einem Fenster 66 verschlossen ist.

Der Lichtleiter 64 ist soweit in die Bohrung 68 eingeschoben, daß in die etwa sacklochartige, allerdings am Boden offene Aufnahme 62 der erste zylindrische Zapfen 22 passend einschiebbar ist, wie das aus der Schnittdarstellung von Fig. 4 ersichtlich ist. Eine auf dem vom Gehäuse 54 vorstehenden Ende aufgeschobene Tülle 65 des Lichtleiters 64 umrundet ein hier nicht näher bezeichnetes Glasfaserbündel, das mit einer Lichtquelle verbunden ist.

Wie das aus der Schnittdarstellung von Fig. 4 zu entnehmen ist, ist im Gehäuse 54 eine Fokussiereinheit 72 vorgesehen, die einen Stellring 74 aufweist.

Im optischen Pfad hinter der Fokussiereinheit 72 ist ein Bildaufnahmesystem 76 angeordnet. Dieses Bildaufnahmesystem 76 weist bspw. einen in dieser Technologie üblichen CCD-Sensor auf, der das vom Endoskop über die Fokussiereinheit 72 fokussierte Bild in ein elektrisches Signal umwandelt. Das Bildaufnahmesystem 76 ist in einer Hülse 78 aufgenommen, die drehbar im Gehäuse 54 aufgenommen ist. Endseitig ist die Hülse 78 mit einer Tülle 80 versehen, die ein hier nicht näher bezeichnetes Kabel umgibt, das die vom CCD-Sensor erzeugten elektrischen Signale einer Bildverarbeitungseinheit zuspeist. Die Drehbarkeit des Bildaufnahmesystems 76 dient dazu, um das vom Endoskop 12 kommende Bild auf einem Monitor in eine bestimmte Nord-Süd-Ausrichtung zu bringen bzw. aufzurichten, falls das Endoskop 12 gedreht wird.

Im Gehäuse 54 ist im Bereich des Kupplungsendes 55 ein radial zur Kupplungsrichtung 50 verschiebbarer Riegel 62 aufgenommen.

Der Riegel 82 hat etwa die Form einer zweizinkigen Gabel, deren beide Zinken 86 und 87 am äußeren Ende kreisförmig nach innen gebogen sind, wobei der Krümmungsradius in etwa dem Krümmungsradius der Aufnahme 58 entspricht. Die beiden Zinken 86 und 87 sind über einen außerhalb des Gehäuses gelegenen Bügel 88 miteinander verbunden. Zwischen Bügel 88 und Gehäuse 54 ist eine Druckfeder 90 angeordnet, die den Riegel 82 radial nach außen drückt. In diesem Zustand ragen die äußeren Enden der Zinken 86 und 87 etwas in die Aufnahme 58 hinein, wie das aus der Schnittdarstellung von Fig. 2 ersichtlich ist.

Zum Kuppeln werden beide Bauelemente, also Endoskop 12 und Kameramodul 14 von je einer Hand ergriffen und die Zapfen 22 bzw. 30 des Endoskops 12 werden in die entsprechenden Aufnahmen 58 und 62 hineingesteckt.

Dabei trifft der konische Abschnitt 46 des zweiten Zapfens 30 auf die in die Aufnahme 58 hineinragenden Enden der Zinken 86 und 87 und verschieben diese radial nach außen, wobei der Riegel 82, in der Darstellung von Fig. 2, etwas von oben nach unten bewegt wird.

Wurde der zweite Zapfen 30 soweit in die Aufnahme 58 hineingeschoben, bis die Zinken 86 und 87 auf Höhe der Ringnut 54 zum Liegen kommen, schnappen diese in die Ringnut 44 hinein, was durch die Kraft der Feder 90 unterstützt wird.

In dieser Position ist nunmehr die Kupplung geschlossen, d.h. das Kupplungssystem 10 ist verkuppelt und mechanisch verriegelt. In diesem Zustand liegen die Fenster 37 von zweitem Zapfen 30 und Fenster 60 im Boden der Ausnehmung 58 deckungsgleich aufeinander, so daß eine bildleitende Kupplung geschaffen ist. Das Fenster 29 des ersten Zapfens 22 kommt vor dem Fenster 60 des Lichtleiters 64 zum Liegen, so daß auch eine lichtleitende Kupplung geschaffen ist.

Zum Lösen der Kupplung muß lediglich der Riegel 82 axial nach innen gerückt werden, wodurch die gekrümmten Enden der Zinken 86 und 87 aus der Ringnut 44 austreten, so daß das Endoskop 12 vom Kameramodul 14 abgezogen werden kann.

Aus der Draufsicht von Fig. 3 ist zu erkennen, daß im Gehäuse 18 des Endoskopes und dann auch entsprechend im Gehäuse 54 des Kameramoduls eine weitere Kupplungsstelle 92 vorgesehen sein kann, bspw. in Form eines Insufflations- oder Spülkanales.

Ferner kann am Gehäuse 18 des Endoskopes 12 ein Codierelement 94 vorgesehen sein, das von dem Kameramodul 14 gelesen werden kann, so daß erkannt werden kann, mit welchem Endoskop 12 gekoppelt wurde.

## Patentansprüche

1. Kupplung (10) zum mechanischen, lichtleitenden und bildleitenden Verbinden eines Endoskopes (12) mit einem Kameramodul (14), bestehend aus zwei Kupplungsteilen, nämlich aus einem Kupplungsende (20) des Endoskopes (12) und einem Kupplungsende (55) des Kameramoduls (14), wobei das Endoskop (12) einen langen zylindrischen Schaft (16) von etwa gleichbleibendem Durchmesser aufweist, wobei der Schaft (16) ein Bildleitsystem (36) und ein Lichtleitsystem (28) aufnimmt, wobei am Kupplungsende (20) des Endoskopes (12) ein erster zylindrischer vorstehender Zapfen (22) bestimmten Durchmessers (24) und bestimmter Länge (26) in Kupplungsrichtung (50) angebracht ist, in dessen Innerem ein proximaler Endabschnitt des Lichtleitsystems (28) aufgenommen ist,
und am Kupplungsende (20) des Endoskopes (12) ein zweiter, etwa zylindrischer vorstehender Zapfen (30) in Kupplungsrichtung (50) angebracht ist, dessen Länge (34) und Durchmesser (32) größer als die Länge (36) und der Durchmesser (24) des ersten Zapfens (22) sind, wobei im Inneren des zweite Zapfens (30) ein proximaler Endabschnitt des Bildleitsystems (36) aufgenommen ist,
wobei der zweite Zapfen (30) mit einer am Kameramodul (14) angeordneten Verriegelung zum mechanischen Verriegeln der Kupplung (10) zusammenwirkt,
wobei sich erster und zweiter Zapfen (22, 30) im Abstand nebeneinander erstrecken,
wobei am Kupplungsende (55) des Kameramoduls (14) den beiden Zapfen (22, 30) entsprechende komplementäre Aufnahmen (62, 58) vorgesehen sind, in die die Zapfen (22, 30) eindringen,
und wobei ein Boden der Aufnahme (58), in die der zweite Zapfen (30) eindringt, optisch mit dem Bildaufnahmesystem (76) des Kameramoduls (14) verbunden ist und die Aufnahme (62), in die der kürzere erste Zapfen (62) aufnehmbar ist, einen Lichtleiter (64) aufweist.

2. Kupplung nach Anspruch 1, **dadurch gekennzeichnet, daß** die beiden Zapfen (22, 30) von einem Gehäuse (18) vorstehen, dessen Querschnittskontur einer um die beiden Zapfen (22, 30) unterschiedlichen Durchmessers (24, 32) umlaufenden Ortslinie (38) entspricht.

3. Kupplung nach Anspruch 1 oder 2, **dadurch gekennzeichnet, daß** das Kameramodul (14) im Kupplungsbereich ein Gehäuse (54) aufweist, dessen Querschnittskontur einer um die beiden Aufnahmen (58, 62) unterschiedlichen Durchmessers umlaufenden Ortslinie (56) entspricht.

4. Kupplung nach einem der Ansprüche 1 bis 3, **dadurch gekennzeichnet, daß** die Verriegelung als quer zur Kupplungsrichtung (50) verschiebbarer Riegel (82) ausgebildet ist, der in eine Aussparung am zweiten Zapfen (30) einrückbar ist.

5. Kupplung nach einem der Ansprüche 4, **dadurch gekennzeichnet, daß** der Riegel (82) durch die Kraft einer Feder (90) beaufschlagt ist, und von dieser etwas in die zweite Aufnahme (58) für den zweiten Zapfen (30) radial hineingedrückt wird.

6. Kupplung nach einem der Ansprüche 1 bis 5, dadurch gekennkeichnet, daß der zweite Zapfen (30) endseitig einen konischen Abschnitt (46) aufweist, der von einer Hinterschneidung (48) gefolgt ist.

7. Kupplung nach Anspruch 6, **dadurch gekennzeichnet, daß** die Hinterschneidung (48) als Ringnut (44) ausgebildet ist.

8. Kupplung nach einem der Ansprüche 4 oder 5, **dadurch gekennzeichnet, daß** der Riegel (82) über das Gehäuse (54) des Kameramoduls (14) vorsteht.

9. Kupplung nach einem der Ansprüche 1 bis 8, **dadurch gekennzeichnet, daß** im Gehäuse (18) des Endoskopes (12), von dem die Zapfen (22, 30) vorstehen, ein Zwischenabbildungssystem (52) aufgenommen ist.

10. Kupplung nach einem der Ansprüche 1 bis 9, **dadurch gekennzeichnet, daß** das Kameramodul (14) ein Bildaufnahmesystem (76) mit mindestens einem CCD-Sensor aufweist.

11. Kupplung nach einem der Ansprüche 1 bis 10, **dadurch gekennzeichnet, daß** das Kameramodul (14) eine Fokussiereinheit (72) mit einem am Gehäuse (54) angeordneten Stellglied (74) aufweist.

12. Kupplung nach Anspruch 11, **dadurch gekennzeichnet, daß** die Fokussiereinheit (72) über eine Verstellung von Linsenelementen und/oder des CCD-Sensors die Scharfeinstellung des Bildes bewirkt.

13. Kupplung nach einem der Ansprüche 10 bis 12, **dadurch gekennzeichnet, daß** das Bildaufnahmesystem (76) zur Bildaufrichtung drehbar im Gehäuse (54) aufgenommen ist.

14. Kupplung nach Anspruch 13, **dadurch gekennzeichnet, daß** die Bildaufrichtung über eine Drehbarkeit des CCD-Sensors oder über eine Zwischenoptik, z.B. über K-Prismen, erfolgt.

15. Kupplung nach einem der Ansprüche 1 bis 14, **dadurch gekennzeichnet, daß** der zweite Zapfen (30) und die diesen aufnehmende zweite Aufnahme (58) jeweils ein Fenster (37, 60) aufweisen.

16. Kupplung nach einem der Ansprüche 1 bis 15, **dadurch gekennzeichnet, daß** sowohl im Endoskop (12) als auch im Kameramodul (14) zumindest eine weitere Kopplungsstelle (92) für einen Insufflations- oder Spülkanal vorgesehen ist.

17. Kupplung nach einem der Ansprüche 1 bis 16, **dadurch gekennzeichnet, daß** am Gehäuse (18) des Endoskopes (12) ein endoskopspezifisches Codierelement (94) vorgesehen ist, das mit einem Leseelement am Kameramodul (14) gekoppelt wird.

## Claims

1. Coupling (10) for mechanical, light-guiding, and image-guiding connection of an endoscope (12) to a camera module (14), comprising two coupling elements, namely a coupling end (20) of the endoscope (12) and a coupling end (55) of the camera module (14), the endoscope (12) having a long cylindrical shaft (16) of approximately constant diameter, the shaft (16) receiving an image guiding system (36) and a light guiding system (28), a first protruding cylindrical stem (22) of specific diameter (24) and of specific length (26), is disposed at the coupling end (20) of the endoscope (12), in whose interior is received a proximal end segment of the light guiding system (28), and a second protruding approximately cylindrical stem (30) is disposed in the coupling direction (50), whose length (34) and diameter (32) are greater than the length (26) and diameter (24) of the first stem (22), a proximal end segment of the image-guiding system (36) being received in the interior of the second stem,
with the second stem (30) coacting with an interlock system arranged on the camera module (14) for mechanically interlocking of the coupling (10);
with the first and second stems (22, 30) extending at a distance next to one another; and
with complementary receptacles (62, 58) corresponding to the two stems (22, 30), into which the stems (22, 30) penetrate, being provided at the coupling end (55) of the camera module (14), and
wherein a base of the receptacle (58) into which the second stem (30) penetrates being optically connected to the image sensing system (76) of the camera module (14), and the receptacle (62) in which the shorter first stem (62) is receivable having a light guide (64).

2. Coupling of claim 1, **characterized in that** the two stems (22, 30) protrude from a housing (18), whose cross-sectional contour corresponds to a locus curve (38) running around the two stems (22, 30) of different diameters (24, 32).

3. Coupling of claims 1 or 2, **characterized in that** the camera module (14) has in the coupling region a housing (54) whose cross-sectional contour corresponds to a locus curve (56) running around the two receptacles (58, 62) of different diameters.

4. Coupling of anyone of claims 1 through 3, **characterized in that** the interlock system is configured as a locking element (82), displaceable transversely to the coupling direction (50), that can be engaged into a recess on the second stem (30).

5. Coupling of claim 4, **characterized in that** the locking element (82) is acted upon by the force of a spring (90), and is radially pressed by it slightly into the second receptacle (58) for the second stem (30).

6. Coupling of anyone of claims 1 through 5, **characterized in that** the second stem (30) has on the end a conical segment (46) that is followed by an undercut (48).

7. Coupling of claim 6, **characterized in that** the undercut (48) is configured as an annular groove (44).

8. Coupling of anyone of claims 4 or 5, **characterized in that** the locking element (82) projects beyond the housing (54) of the camera module (14).

9. Coupling of anyone of claims 1 through 8, **characterized in that** an intermediate imaging system (52) is received in the housing (18) of the endoscope (12) from which the stems (22, 30) protrude.

10. Coupling of anyone of claims 1 through 9, **characterized in that** the camera module (14) has an image sensing system (76) with at least one CCD sensor.

11. Coupling of anyone of claims 1 through 10, **characterized in that** the camera module (14) has a focusing unit (72) with an adjusting member (74) arranged on the housing.

12. Coupling of claim 11, **characterized in that** the focusing unit (72) effects focusing of the image by shifting lens elements and/or the CCD sensor.

13. Coupling of anyone of claims 10 through 12, **characterized in that** for an image erection, the image sensing system (76) is received rotatably in the housing (54).

14. Coupling of claim 13, **characterized in that** the image erection is accomplished via a rotation capability of the CCD sensor or via an intermediate optical system, i.e. via K prisms.

15. Coupling of anyone of claims 1 through 14, **characterized in that** the second stem (30) and the second receptacle (58) receiving it each have a window (37, 60).

16. Coupling of anyone of claims 1 through 15, **characterized in that** at least one further coupling point (92) for an insufflation duct or a flushing duct is provided both in the endoscope (12) and in the camera module (14).

17. Coupling of anyone of claims 1 through 16, **characterized in that** an endoscope-specific coding element (94), which is coupled to a read element on the camera module (14), is provided on the housing (18) of the endoscope (12).

## Revendications

1. Accouplement (10) pour la liaison mécanique, conductrice de lumière et d'image d'un endoscope (12) avec un module de caméra (14), composé de deux parties d'accouplement, à savoir d'une extrémité d'accouplement (20) de l'endoscope (12) et d'une extrémité d'accouplement (55) du module de caméra (14), l'endoscope (12) comportant une longue tige cylindrique (16) d'un diamètre presque constant, la tige (16) logeant un système de conduction de l'image (36) et un système photoconducteur (28), un premier tourillon cylindrique en saillie (22), d'un diamètre précis (24) et d'une longueur précise (26), étant disposé à l'extrémité d'accouplement (20) de l'endoscope (12) dans le sens d'accouplement (50), tourillon à l'intérieur duquel est logée une section finale proximale du système photoconducteur (28),
et un deuxième tourillon à peu près cylindrique en saillie (30) étant disposé à l'extrémité d'accouplement (20) de l'endoscope (12) dans le sens d'accouplement (50), tourillon dont la longueur (34) et le diamètre (32) sont supérieurs à la longueur (36) et au diamètre (24) du premier tourillon (22), une section finale proximale du système conducteur d'image (36) étant logée à l'intérieur du deuxième tourillon (30),
le deuxième tourillon (30) concourant avec un dispositif de verrouillage disposé sur le module de caméra (14) pour le verrouillage mécanique de l'accouplement (10),
le premier et le deuxième tourillons (22, 30) s'étendant l'un à côté de l'autre, à distance,
des logements (62, 58) complémentaires, correspondant aux deux tourillons (22, 30), étant prévus à l'extrémité d'accouplement (55) du module de caméra (14), logements dans lesquels pénètrent les tourillons (22, 30),
et un fond du logement (58), dans lequel le deuxième tourillon (30) pénètre, étant optiquement relié au système de prise de vue (76) du module de caméra (14) et le logement (62), dans lequel le premier tourillon plus court (26) peut être logé, comprenant un conducteur de lumière (64).

2. Accouplement selon la revendication 1, **caractérisé en ce que** les deux tourillons (22, 30) dépassent d'un boîtier (18) dont le contour de coupe transversale correspond à un lieu géométrique (38) entourant les deux tourillons (22, 30) de diamètres différents (24, 32).

3. Accouplement selon la revendication 1 ou 2, **caractérisé en ce que** le module de caméra (14) comprend, dans la zone d'accouplement, un boîtier (54) dont le contour de section transversale correspond à un lieu géométrique (54) entourant les deux logements (58, 62) de diamètre différent.

4. Accouplement selon l'une des revendications 1 à 3, **caractérisé en ce que** le verrouillage est configuré en tant que barre mobile (82) transversalement au sens d'accouplement (50), barre qui peut être enfoncée dans un évidement situé au niveau du deuxième tourillon (30).

5. Accouplement selon la revendication 4, **caractérisé en ce que** la barre (82) est alimentée par la force d'un ressort (90) et est quelque peu enfoncée, de manière radiale, par ce dernier dans le deuxième logement (58) du deuxième tourillon (30).

6. Accouplement selon l'une des revendications 1 à 5, **caractérisé en ce que** le deuxième tourillon (30) comprend du côté final une section conique (46) qui est suivie par une contre-dépouille (48).

7. Accouplement selon la revendication 6, **caractérisé en ce que** la contre-dépouille (48) est configurée en tant que rainure annulaire (44).

8. Accouplement selon l'une des revendications 4 ou 5, **caractérisé en ce que** la barre (82) dépasse du boîtier (54) du module de caméra (14).

9. Accouplement selon l'une des revendications 1 à 8, **caractérisé en ce que**, dans le boîtier (18) de l'endoscope (12), à partir duquel les tourillons (22, 30) font saillie, un système d'imagerie intermédiaire (52) est logé.

10. Accouplement selon l'une des revendications 1 à 9, **caractérisé en ce que** le module de caméra (14) comprend un système de prise de vue (76) avec au moins un capteur CCD.

11. Accouplement selon l'une des revendications 1 à 10, **caractérisé en ce que** le module de caméra (14) comprend une unité de focalisation (72) avec un élément de réglage (74) disposé sur le boîtier (54).

12. Accouplement selon la revendication 11, **caractérisé en ce que** l'unité de focalisation (72) induit la mise au point de l'image par un réglage des éléments à lentille et/ou du capteur CCD.

13. Accouplement selon l'une des revendications 10 à 12, **caractérisé en ce que** le système de prise de vue (76) est logé dans le boîtier (54) de manière à pouvoir tourner, pour le redressement de l'image.

14. Accouplement selon la revendication 13, **caractérisé en ce que** le redressement de l'image se fait par le biais d'une rotation du capteur CDD ou par le biais d'un objectif intermédiaire, par exemple par le biais de prismes K.

15. Accouplement selon l'une des revendications 1 à 14, **caractérisé en ce que** le deuxième tourillon (30) et le deuxième logement (58) recevant ce dernier présentent respectivement une fenêtre (37, 60).

16. Accouplement selon l'une des revendications 1 à 15, **caractérisé en ce qu'**à la fois dans l'endoscope (12) et dans le module de caméra (14), on a prévu au moins un autre emplacement d'accouplement (92) pour un canal d'insufflation ou de rinçage.

17. Accouplement selon l'une des revendications 1 à 16, **caractérisé en ce qu'**est prévu un élément de codage (94), spécifique à l'endoscope, au niveau du boîtier (18) de l'endoscope (12), lequel élément est couplé à un appareil de lecture situé au niveau du module de caméra (14).
